Europäisches Patentamt

European Patent Office

Office européen des brevets

(19)

(11) Publication number: **0 264 164**
A1

(12) **EUROPEAN PATENT APPLICATION**

(21) Application number: **87302729.6**

(22) Date of filing: **30.03.87**

(51) Int. Cl.³: **A 61 F 9/06**

(30) Priority: **09.10.86 GB 8624288**

(43) Date of publication of application:
**20.04.88 Bulletin 88/16**

(84) Designated Contracting States:
**AT BE CH DE ES FR IT LI NL SE**

(71) Applicant: **Henry Factory Company Limited**
**No. 3, Lane 21, Sec. 2, Jong-lang Road**
**Tuu Cherng Hsiang Taipei Hsien(TW)**

(72) Inventor: **Li, Edward K.**
**No. 79 Lane 394 Taichung Road**
**Taichung City(TW)**

(74) Representative: **Arthur, Bryan Edward**
**Withers & Rogers 4 Dyer's Buildings Holborn**
**London EC1N 2JT(GB)**

(54) **Welding maskholder.**

(57) A special welding apparatus comprises a welding pliers (25) and a head shield (21), which may be combined together by means of an engaging groove, a pressure ball, a fixed ring (30), a fixed hook, and a hook seat, or may be separated from each other. The colored goggle assembly (22) has a sliding mechanism; upon a key (27) on the handle (28) of head shield (21) being pressed down, the colored goggle (22) will slide away from the window (23), and when the key (27) is released, the colored goggle (22) will be moved back over the window (23).

FIG·1

Croydon Printing Company Ltd

EP 0 264 164 A1

WELDING MASKHOLDER

This invention relates to a special welding apparatus, particularly a welding shield and pliers with a movable goggle; in other words, the colored goggle can be moved back or away from the transparent goggle window.

In the modern industry, a product not only should have a better quality, but also should have a lower cost and price. In order to achieve the aforesaid goal, a better apparatus or tool is essential to the modern industry, i.e., a tool should be practical, durable, of precision, and should have more functions so as to lower the cost, to protect the user, and to increase the working efficiency.

The welding process is often required in many aspects in the modern industry. The welding quality and efficiency are directly related to the manufacturing costs. In electric welding process, the most part thereof is done manually ; it is certainly believed that the manual welding operation will permanently be used in the industries as a result of many reasons such as some parts having no regular specifications, the factories or working sites having different facilities and scales; in other words, a full automatic welding operation can not be adopted in all factories. Therefore, it is considered being urgently required to develop a welding apparatus for the modern industry so as to protect the welder's safety, to lower the cost and to elevate the quality of products welded, and to elevate the welding efficiency.

The existing electric welding head shields may be classified into two kinds, i.e., a hand-holding type and a head-wearing type.   The head-wearing type is further classified into three classes: a fixed colored goggle type, a movable colored goggle type ( i.e.,the colored goggle on the window able to be opened or closed ), a type with helmet.  When using the head-wearing type of head shield, the welder has to shake his head downwards to cause the shield to fall down from the top portion so as to let the window stop before the eyes for protection purpose.  The welder will have a spare hand to hold the working piece, therefore, that head-wearing type of shield can, under certain circumstances, increase the working efficiency, and is used by many factories and welders in the developed countries.

According to the welding experiences, an intense welding arc and a toxic smoke will simultaneously be generated during welding operation.  Since most of the conventional head shields are designed into a conic pot shape, in which the smoke entered is not easy to be exhausted; therefore, the face of the welder will accumulate a lot of smoke, which would cause some occupational diseases, such as bronchitis, pink eye, spitting hack, etc. The welder is susceptible to suffering from such diseases when working at a place where has a poor ventilation.  Moreover, if the welder does not wear a normal helmet, the situation may become worse. If the welder is required to wear a helmet during welding, a regular and professional helmet  has to be used. A helmet usually called by the welders as a melon rind type of helmet without visor should not be used because of it being unable to protect the welder's ears and nose in case of a harmful stuffs falling on the head.

When using a hand-holding type of head shield, the welder would be rather difficult to have one spare hand to hold the working piece, while his other hand is used to make spot welding. Usually, the welder closes his eyes to prevent the intense welding arc from radiating into the eyes, and to let his face expose to the intense welding arc. Experience has proved that the welder after long time exposing to such intense welding arc would suffer from a dry skin face, the skin around the eyes becoming swelling, or a skin-peeling off from the face, an eye disease, or a skin cancer. Some one might say that there are many welders still working in the conventional manner without trouble ; in fact, they are either not aware of the danger because of lacking such knowledge, or having no choice.

A real example is described for reference. There is a factor located in a certain processing zone in Taichung area, which is specially doing mechanical body work for their customers. One department of the factory one day accepted an order to make a lathe scrap tank. The material for the tank was a steel sheet of 1 m/m thick. After being measured and cut, the steel sheet is bent into form required; then, the side board and pipe joints thereof were added. Since it was a small order with irregular specifications and lower processing price, there was no particular working clamps being designed and made; further, the technicians of that factory were used to conduct welding with the hand-holding type of head shields ( in fact, most of the welders in Taiwan use that kind of head shield ). To make the scrap tank as mentioned above, the various parts thereof have to be assembled together in position first by means of spot welding; then, a regular leak-proof welding was conducted, following by finishing and hole-drilling processes. During the spot welding process, one hand of the welder has to hold the

welding pliers, while the other hand held the working piece; therefore, the welder has to close his eyes to do the welding work by means of experience.  As a result of the radiation by the intense welding arc, it was discovered by the inventor that all of the welders suffered from serious skin-peeling off; some of them even needed to see a doctor for their swelling eye skins.  It is doubtful whether some of them suffered from skin cancer.  Many of the similar aforesaid cases were discovered by the inventor during his working travelling, but it can not fully described herein because of limited pages.

At the present time, efficiency is particularly important in doing everything; naturally, the welding business is also under serious competition; the welders seem to have less or no time to pre-fix the working piece before putting on the head shield and starting to weld the working piece.  Some one might say why the welders do not use the head-wearing type of shield to avoid the aforesaid injuries to happen; in the real working site, the welders usually do many things one after another, such as handling the materials, welding, moving articles from one place to another, or climbing up or down around a big mechanical structure.  In that case, the head shield is often slid down to block the vision of wearer.  The welders may put on or remove the head shield as the case may be, but they may feel it being more inconvenient to them in terms of working efficiency.  In fact, the conventional head-wearing type of head shield is quite not good for use.  Another instance was personally seen by the inventor during the period of staying in San Francisco. The inventor was once working in a shipyard as a welder. According to the regulations of the insurance company, every worker working in a ship must wear a helmet; the helmet for the welder is special type that has no visor, and the American welders called it a melon-rind type of helmet. One day, the inventor saw a piece of wood falling on one worker's ear, which was seriously injured, and that worker was a welder working on a hanging scaffold. The inventor also believes the head shield of that type of special helmet wasn't a good head shield. If it is tightly put on the head, the wearer would have a headache. If it is loosely put on the head, the window is often not fallen on the correct position upon the head shield being pulled down; further, the head shield fallen down often swinging laterally. The inventor also inquired the point of view of the American welders on that type of helmet, they all shared with my opinion. Consequently, the inventor brought a hand-holding type of head shield to the working site so as to use it the helmet with visor. Unfortunately, one day the inventor had to hold the working piece with one hand and to hold the welding pliers with the other hand. In that case, the inventor had to close his eyes to perform the welding operation. After working for one day, he felt a serious dry feeling on the face, and had a serious swelling around the eyes the next morning, and then had a serious skin-peeling off after several days. The insurance company said that no damages could be claimed because of the insurant not observing the regulations to wear a head shield.

Summing up the aforesaid facts, it is evident that the current two types of head shields all have their drawbacks. The inventor has over 20 years of welding experiences ; in that period, no one has paid attention to that drawbacks. Since the inventor is a business man, and has understood that the manufacturing cost and the quality of a product would directly be related to the success of an enterprise. If an enterprise is provided with the best tools, it would be instrumental to its success.

In view of the aforesaid facts, the inventor has developed an improved welding shield, which has been tested and proved being practical in welding operation, and is named as "WELDING MASKHOLDER".

In this welding apparatus, the pliers and the head shield can easily be assembled together or disassembled by means of an engaging groove, a pressure ball, a fixed ring, a fixed hook and a hook seat. The colored goggle is furnished with a sliding mechanism. Upon the key on the handle being pressed, the colored goggle will be slid away from the window so as to let the welder see, via the transparent plain glass, the working piece and the position or angle of the welding rod. Upon the key being released, the colored goggle will be slid over the window so as to let the welder conduct spot welding or line welding. As soon as the two working piece being welded together, the welder will have a spare hand to hold the working piece or to act otherwise. Since the present invention can be used as the welding pliers, a head shield and a plain optical glass to protect the welder's face, it will also be able to elevate the working efficiency.

In view of explaining more clearly the characteristics of the invention, but merely as an example and without the intent of limitation, a preferred form of embodiment will be described hereinafter with reference to the appended drawings in which :

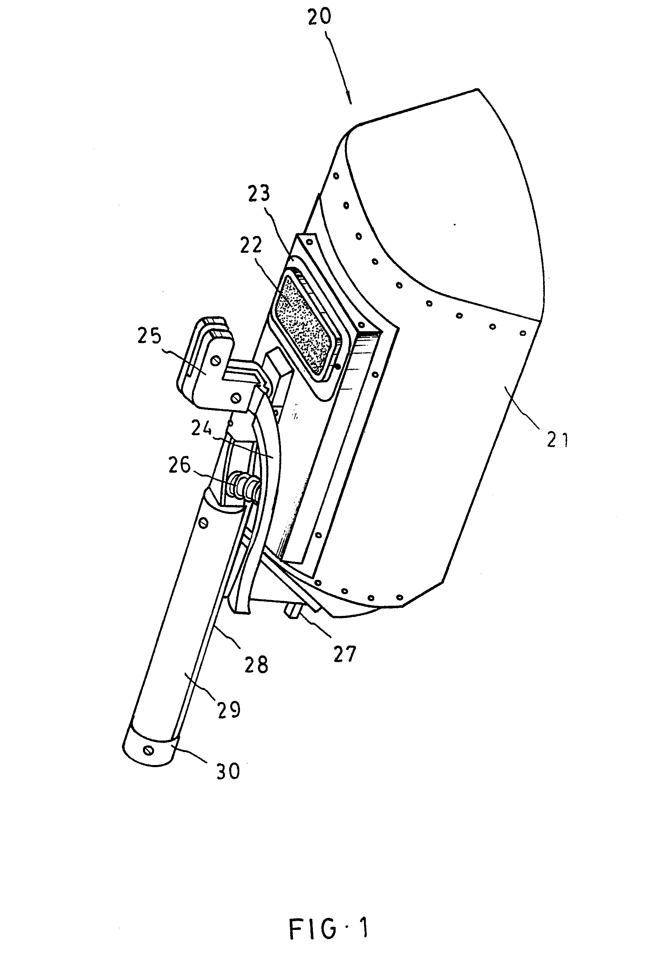

Fig. 1 is a perspective view of the present invention, showing the head shield and the welding pliers being assembled

together.

Fig. 2 is a rear view of Fig. 1, showing the control mechanism of the goggle window.

Fig. 3 illustrates the operation view of the control mechanism of the goggle window, particularly showing the goggle being pushed upwards to close the window.

Fig. 4 is a side view of the present invention, showing the goggle being pulled away from the goggle window.

Fig. 5 is the right side view of Fig. 1.

Fig. 6 is a top view of Fig. 1.

Fig. 7 is bottom view of Fig. 1.

Fig. 8 is a front view of the goggle window control mechanism, showing the goggle window control key being set at the upper dead point.

Fig. 9 is a side view of the goggle window control mechanism, showing the goggle window control key being set at the lower dead point.

Fig. 10 is a rear view of the welding pliers.

Fig. 11 is a sectional view taken along line 11-11 in Fig. 10.

Fig. 12 is a side view of the welding pliers.

Fig. 13 is a top view of the welding pliers.

Fig. 14 is a bottom view of the welding pliers.

As shown in the drawings attached, the welding shield and pliers with a movable goggle 20 according to the present invention comprises a head shield 21 made of fibers, a colored goggle 22, a lower transparent frame 23, a welding plier handle 24, a zigzag-rule type of welding pliers 25, a spring 26, a window

control key 27, a shield handle 28, a welding plier handle 29, a fixing ring 30, screws 31, an upper transparent frame 32, a pulley 33 , a steel cord 34, a fixed ring 35, a goggle sliding rod 36, a bearing 37, a goggle frame 38, a nut 39, a control key sliding rod 40, a sliding rod seat 41, a window 42, a wire hole 43, a fixed hook 44 , a hook seat 45, a pressure ball 46, a sliding groove 47, movable balls 48, an engaging groove 49, a conductor 50, a shaft 51 and a protective layer 52.

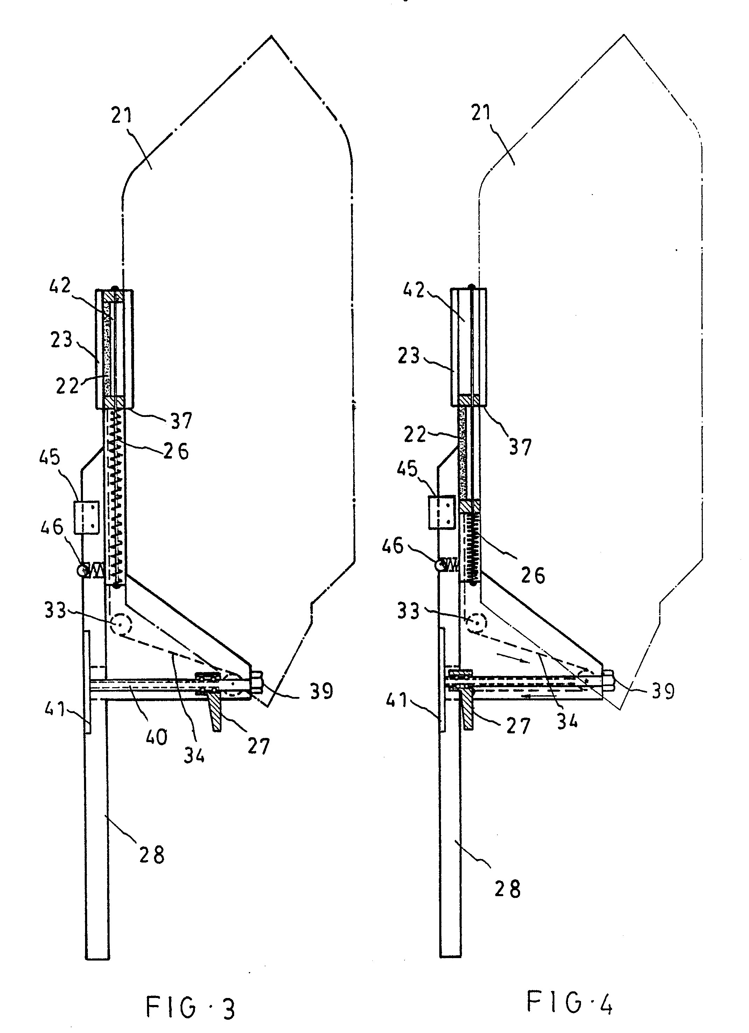

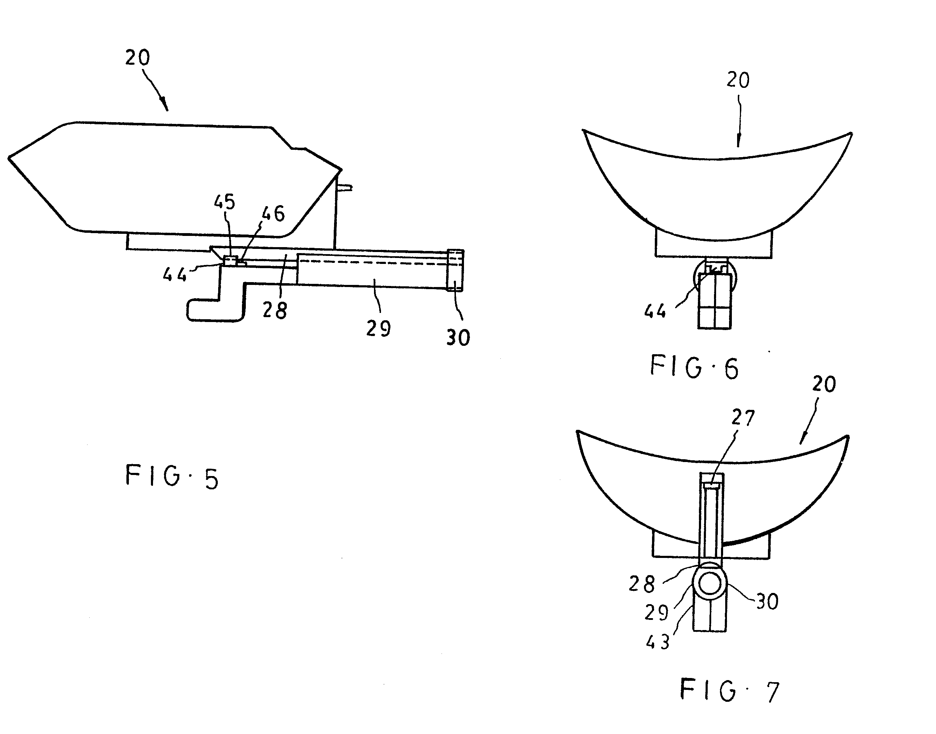

Upon using the welding shield and pliers with movable goggle, one hand of the user should hold the working piece, while the other hand will have the welding pliers 25 and the head shield 21 assembled together ( as shown in Figs.5, 6 and 7 ), while the other hand should press down the window control key 27 on the shield handle 28 so as to have the goggle 22 slid away from the goggle window 42 as shown in Fig. 4, and to let the user clearly see, via the window 42, the working piece to be pointed at the spot with the welding rod; then, release the goggle window control key 27, and let the welding rod touch the working piece for either spot welding or line welding. Since the welding pliers 25 and the head shield 21 are combined together to be held with one hand, the other hand of the user can hold the working piece or do other work. Since the user's face is protected with the head shield 21, the welding arc would not injure the face of a welding person, who can also watch the welding condition of the working piece via the colored goggle 22 so as to maintain a better welding quality. In the past, a welding person has to close his eyes to make a spot welding for testing a stainless steel working piece; in that case, the working piece is susceptible to being burnt through or being

not welded firmly; occasionally, the welding arc might be discontinued or the welding rod might be stuck on the working piece. As a result of the stainless steel, the cooled welding scales might automatically fly .randomly out of the working piece to hit the eye of a welding person. Upon one welding rod being used up and using the next welding rod, the welding scales on the last welded part on the working piece have to be removed with a tool so as to maintain a better welding quality. In fact, few welders wear the head shield; some of them might wear a pair of eye glasses, but the high temperature welding scales might fly to the face to cause an injury. Therefore, many welders are afraid of welding the stainless working piece. The aforesaid care can be removed by using the present invention, a welding shield and pliers with a movable goggle 20.

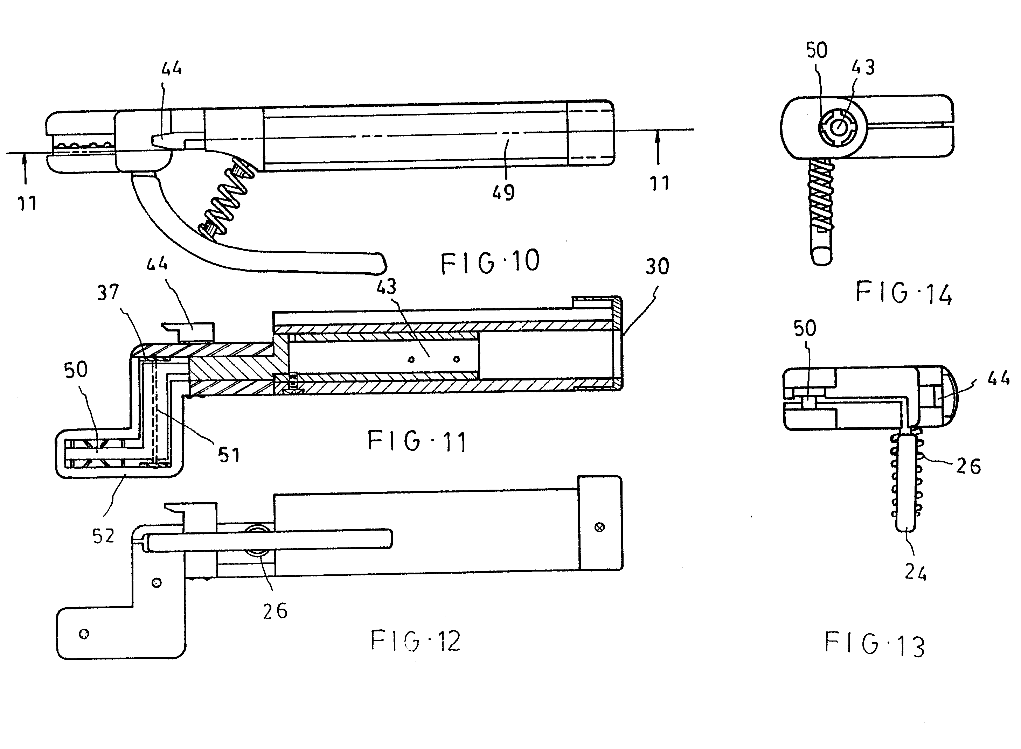

The welding shield and pliers with a movable goggle 20 comprises an engaging groove 49, a fixing ring 30, a fixed hook 44, a hook seat 45, and a pressure ball 46. Referring to Figs. 3-7 and 10-14, it is shown that the two parts after being engaged together would not be separated automatically under normal condition because of the action of the pressure ball 46. A sample of the present invention made by the inventor was tested for about 500 times of engaging and disengaging, and was also tested by hitting the welding shield with force until the pressure ball 46 scratching a vivid scratch on the fixed hook 44 ; the whole welding shield never showed any deformation or other abnormal state. It is evident that a tool like the present invention can withstand the rough use by a rough welder; in other words, the welding shield according to the present invention can, under normal condition, be used for a considerable long time.

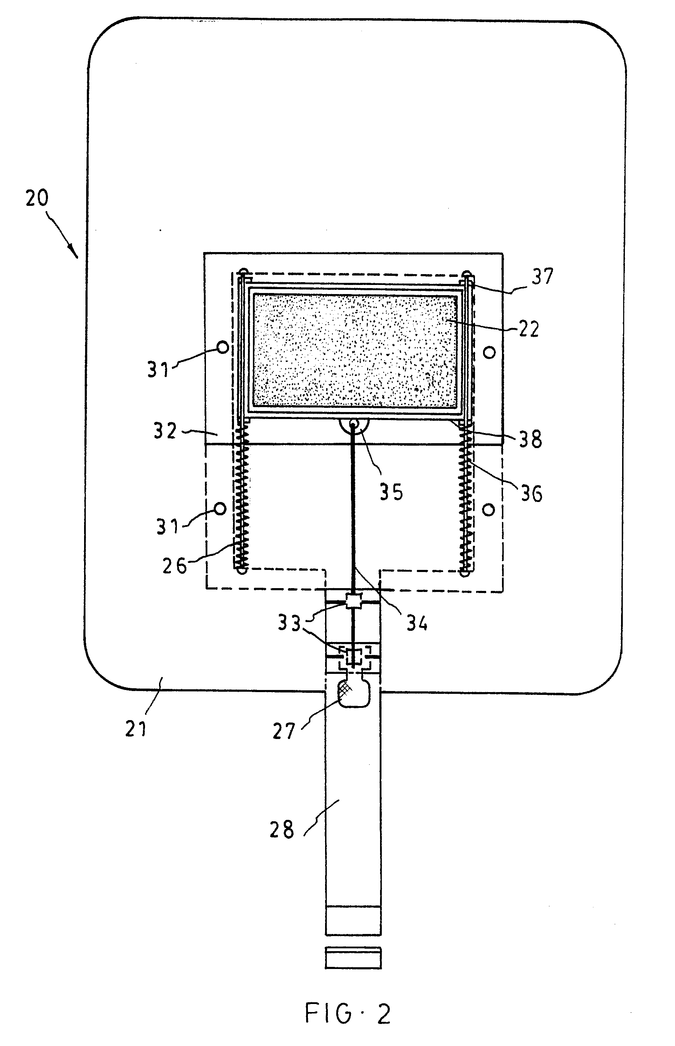

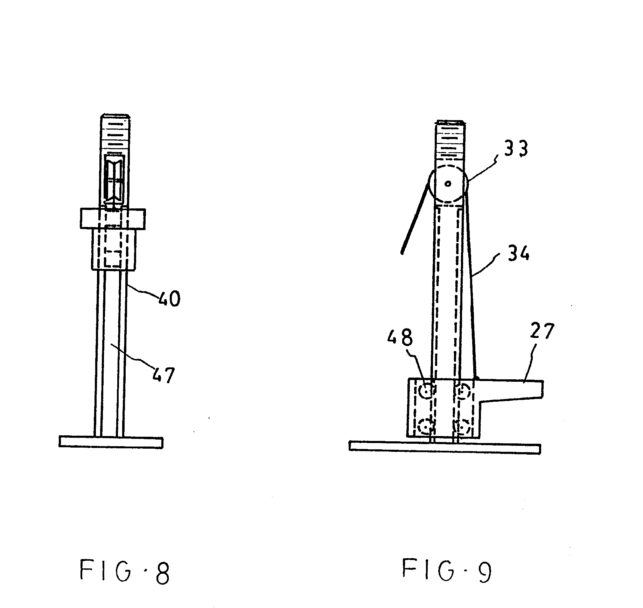

The goggle window control mechanism of the welding shield and pliers with a movable goggle 20 is operated by means of a number parts which include a control key 27 , a movable ball 46, a sliding groove 47, a sliding rod 40, a goggle sliding rod 36, a steel cord 34, and a pulley 33, all of which are driven with a spring as shown in Figs. 2, 3, 4, 8 and 9 . During the aforesaid test of 500 times, it has been proved that the whole sliding system can withstand the tests.

The user of the welding shield and pliers with a movable goggle according to the present invention needs little training, and everybody can use it after a very short time of practice. It is strong and durable for long time use. Upon the colored goggle being moved away from the window, the shield is just like a general shield with welding pliers; upon the colored goggle being mounted over the window, the two parts are combined together; in other words, the present invention can be used as either a colored goggle or a plain optical glass. When using the shield according to the present invention, the user will have a spare hand to hold the working piece or to do other motion. Upon pressing down the control key, the colored goggle will be moved away from the window so as to see the working piece and the position of the welding rod through the window; upon the control key being released, the colored goggle will be slid over the window to let the user watch the welding state without injuring his eyes and face. The inventor deems that the present invention is a valuable tool to the industry.

The aforesaid embodiment is used for describing the objects, the features and functions of the present invention.

0264164

Any person skilled in the art may make a change or modification to the aforesaid embodiment in accordance with the aforesaid description without being deemed to deviate from the spirit and scope of the present invention; therefore, the scope of the present invention may only be limited by the claims attached.

CLAIMS

1. A special welding apparatus characterized in that the welding pliers and the head shield are combined into one unit, and said pliers and said shield can also be separated from each other as a discrete unit; and said head shield and welding pliers being combined together by means of an engaging groove, a fixed ring, a fixed hook, a hook seat, and a pressure ball; and said welding pliers being a zigzag-rule type and comprising a handle with a groove, said fixed ring, said fixed hook, a protective layer, a spring and a conductor; and said head shield comprising a window, a fiber head shield, and said handle; and the control mechanism of the window comprising a sliding rod, a sliding groove, a pulley, a movable ball, a steel cord, a spring, a key, a colored goggle, and two layers of transparent glasses; and upon said key being pressed down, and said steel cord pulling the frame of said colored goggle downwards along the goggle sliding rod because of the tension of said spring being overcome and the transparent widhow being exposed; and upon said key being released, said colored goggle being slid over said window as a result of the spring force.

2. A special welding apparatus as claimed in claim 1, wherein the portion of said head shield above said welding pliers is designed to form into an angle relative to the front face of said shield.

3. A special welding apparatus as claimed in claim 1, wherein, upon said key being pressed down, the welder can see the working piece through said transparent glass under full protection to his eyes and face; and upon said being released, the welder can see the welding portion and the welding rod without being injured by the welding flame.

0264164

4. A welding maskholder constructed and arranged substantially as herein described with reference to any of the figures of the drawings.

FIG·1

FIG · 2

0264164

FIG·3    FIG·4

FIG·5

FIG·6

FIG·7

FIG·8

FIG·9

0264164

FIG·10

FIG·11

FIG·12

FIG·14

FIG·13

0264164

# EUROPEAN SEARCH REPORT

European Patent Office

| | DOCUMENTS CONSIDERED TO BE RELEVANT | | EP 87302729.6 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | AU - B1 - 30 040/77 (STANLEY GEORGE FOSTER)<br>* Totality *<br>-- | 1 | A 61 F 9/06 |
| A | CH - A - 324 887 (ATTILA JANOSFIA)<br>* Totality *<br>-- | 1 | |
| A | CH - A - 131 408 (ROBERT SARAZIN)<br>* Totality *<br>-- | 1 | |
| A | DE - A1 - 2 428 301 (POTT PAUL)<br>-- | | |
| A | US - A - 1 822 308 (NORTON)<br>---- | | |

TECHNICAL FIELDS
SEARCHED (Int Cl 4)

A 61 F 9/00

B 23 K 9/00

The present search report has been drawn up for all claims

| Place of search | Date of completion of the search | Examiner |
|---|---|---|
| VIENNA | 12-11-1987 | HÜTTNER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82